# EUROPEAN PATENT APPLICATION

(11) **EP 3 249 409 A1**
(43) Date of publication of application: **29.11.2017**
(21) Application number: 16382230.7
(22) Date of filing: 25.05.2016
(51) Int. Cl.: G01N 33/60, A61K 51/04, G01N 33/80

(54) **FDG (FLUORODEOXYGLUCOSE (18F))-LABELED RED BLOOD CELLS AS DIAGNOSTIC RADIOPHARMACEUTICALS**

(71) Applicant: CNIC Fundación Centro Nacional de Investigaciones Cardiovasculares Carlos III, 28029 Madrid (ES)
(72) Inventor: MATEO DE CASTRO, Jesús, 28029 Madrid (ES); ESPAÑA PALOMARES, Samuel, 28029 Madrid (ES); RUIZ-CABELLO OSUNA, Jesús María, 28029 Madrid (ES)
(74) Representative: Hoffmann Eitle

(57) **Abstract**

In the present invention, we solve the problem of facilitating blood pool measurements accessible to patients or researchers by using ¹⁸FDG for the *in vitro* intracellular labeling of RBCs (Red blood cells). ¹⁸FDG tracer is available worldwide and as a glucose analogue it may be incorporated by the erythrocytes and trapped intracellularly. In this sense, a blood sample (such as whole peripheral blood) can be extracted or isolated from a subject or from another subject with blood compatibility. The extracted or isolated blood can be then incubated in contact with ¹⁸FDG for a period of time and later be reinjected into the patient. Labeling of RBCs with ¹⁸FDG will expand the use of the PET technique to blood pool imaging with the consequent improvement in the field of clinical diagnosis.

## Description

### FIELD OF THE INVENTION

The present invention refers to new radiopharmaceuticals for *in vivo* diagnosis by using blood pool measurements, in particular to the use of RBCs (Red blood cells) intracellularly labelled with ¹⁸FDG.

### BACGROUND OF THE INVENTION

The images of the distribution of blood in the body with non-invasive techniques have many applications in research and at the clinical level. Currently its use is limited to SPECT and PET techniques. The use of SPECT imaging and single gamma-ray emitter Tc-99m labeled RBC as a blood pool imaging agent in nuclear cardiology is well established. However, this technique has a poor image quality as well as a poor quantitative accuracy. In addition, shortage in Tc-99m production may limit its use in the future as it relies on old nuclear reactors that are about to be closed for its production.

In the other hand, PET imaging allows for accurate quantitative results and improved spatial resolution compared to SPECT. The most established PET tracer for blood pool imaging is carbon monoxide (CO), which has been labeled with positron emitting radionuclei such as ¹¹C or ¹⁵O and has been used as an inhaled radiotracer over the last three decades. Carbon monoxide binds reversibly and with high affinity to the hemo group of hemoglobin, producing carboxyhemoglobin. Thus, the inhalation of radiolabeled CO leads to the *in vivo* radiolabeling of hemoglobin in the erythrocytes. However, this measurement is restricted to those facilities equipped with a cyclotron capable of producing ¹¹C (half-life ~20 min.) or ¹⁵O (half-life ~2 min.). Additionally, as CO is a gas, its use as inhaled radiotracer presents radiation protection issues. In summary, these severe limitations make blood pool measurements rarely accessible to patients or researchers.

The present invention provides a solution to facilitate blood pool measurements accessible to patients or researchers.

### BRIEF DESCRIPTION OF THE INVENTION

In the present invention, we solve the problem of facilitating blood pool measurements accessible to patients or researchers by using ¹⁸FDG for the *in vitro* intracellular labeling of RBCs (Red blood cells). ¹⁸FDG tracer is available worldwide and as a glucose analogue it may be incorporated by the erythrocytes and trapped intracellularly. In this sense, a blood sample (such as whole peripheral blood) can be extracted or isolated from a subject or from another subject with blood compatibility. The extracted or isolated blood can be then incubated in contact with ¹⁸FDG for a period of time and later be reinjected into the patient. Labeling of RBCs with ¹⁸FDG will expand the use of the PET technique to blood pool imaging with the consequent improvement in the field of clinical diagnosis.

### BRIEF DESCRIPTION OF THE FIGURES

**FIG 1****.** Efficiency of the RBC labeling process with ¹⁸FDG at different conditions. The term "Ref." indicates the whole amount of ¹⁸FDG added to the blood samples normalized to 100%. Efficiency studies were performed for different RBCs packing fractions and glucose concentrations (a), different incubation times and washing protocols (b) and different stimuli (c).
**FIG. 2****.** Coronal views of CT and PET (maximum intensity projections) images obtained for one rat (a) and one rabbit (b). PET images were recorded starting 5, 25 and 45 minutes after intravenous injection of FDG-labeled RBCs.
**Fig. 3****.** Time activity curves obtained from rat (a) and rabbit (b) studies. Mean SUV values were computed in 2D ROIs selected in different regions: left ventricle (LV), lung, liver, spleen and kidney.

### DETAILED DESCRIPTION OF THE INVENTION

### Definitions

- As used herein "¹⁸FDG (Fluorodeoxyglucose (18F))" also commonly called fluorodeoxyglucose and abbreviated [18F]FDG, 18F-FDG or FDG, is a radiopharmaceutical used in the medical imaging modality positron emission tomography (PET). Chemically, it is 2-deoxy-2-(18F)fluoro-D-glucose, a glucose analog, with the positron-emitting radioactive isotope fluorine-18 substituted for the normal hydroxyl group at the 2' position in the glucose molecule.
- As used herein "Red Blood Cells (RBCs)" also called erythrocytes, are the most common type of blood cell and the vertebrate organism's principal means of delivering oxygen (O₂) to the body tissues-via blood flow through the circulatory system. RBCs take up oxygen in the lungs or gills and release it into tissues while squeezing through the body's capillaries.
- As used herein "glucose depleted red blood cells" is understood as one or a fraction or cell population of red blood cells obtained after washing of the cells with glucose-free physiological solution to reduce the non-metabolized intracelular glucose concentration below 5 mM, preferably below 1 mM, more preferably below 0.5 mM, favouring the uptake upon ¹⁸FDG addition through the glucose transporters. The concentration of glucose can be determined with standard commercial Glucose Assay Kits.
- As used herein "buffy coat" is understood as the fraction of an anticoagulated blood sample that contains most of the white blood cells and platelets following density gradient centrifugation of the blood.
- As used herein "Red blood cells labelled with ¹⁸FDG (Fluorodeoxyglucose (18F))" is understood as a fraction of red blood cells were the totality or part of the non-metabolized intracelular glucose has been substituted (or replaced) with ¹⁸FDG.
- As used herein "Packed RBCs" is understood as a concentrated preparation of red blood cells that is obtained from whole blood after removing the plasma by centrifugation or by other sedimentation procedures.

### Description

RBCs (Red blood cells), also called erythrocytes, are the most abundant cells in the blood and the vertebrate organism's principal means of delivering oxygen (O₂) to the body tissues-via blood flow through the circulatory system. RBCs take up oxygen in the lungs or gills and release it into tissues while squeezing through the body's capillaries. Mammalian erythrocytes are unique among the vertebrates as they are non-nucleated cells in their mature form. In mammals, erythrocytes also lose all other cellular organelles such as their mitochondria, Golgi apparatus and endoplasmic reticulum. As a result of RBCs not containing mitochondria, these cells use none of the oxygen they transport; instead they produce the energy they need by the glycolysis of glucose and lactic acid fermentation on the resulting pyruvate.

Glucose transporter protein 1, or GLUT1 and SGLT1, are particularly present in RBCs. GLUT1 has a strong affinity for glucose and it ensures that RBCs receive appropriate levels of glucose to perform their normal function. The carriage of glucose to the interior of the RBCs is accomplished by conformational changes within GLUT1. Erythrocyte glucose transporter GLUT1 is an asymmetric transporter having approximately 10-fold-lower affinity for D-glucose, Km ≈ 10-15 mM, at the intracellular side of the membrane for net export than on the extracellular side of the membrane (Km = 1-2 mM) for net import of glucose (zero-trans net flux) at 24°C.

¹⁸FDG is a radiopharmaceutical used in PET (Positron Emission Tomography) imaging. Chemically, it is 2-deoxy-2-(18F)fluoro-D-glucose, a glucose analog, with the positron-emitting radioactive isotope fluorine-18 substituting the hydroxyl group at the 2' position in the glucose molecule. Once intracellularly, glucose and ¹⁸FDG are phosphorylated by the action of hexokinases as the first step toward glycolysis. Generally, once phosphorylated glucose continues along the glycolytic pathway for energy production. ¹⁸FDG, however, becomes effectively trapped intracellularly as ¹⁸FDG-6-Phosphate. The concentration of ¹⁸FDG tracer imaged indicates tissue metabolic activity as it corresponds to the regional glucose uptake. Use of this tracer to explore the possibility of cancer metastasis is the most common type of PET scan in standard medical care (90% of current scans).

In the present invention, we proposed the use of ¹⁸FDG for the *in vitro* intracellular labeling of RBCs (Red blood cells) in order to perform *in vivo* blood pool measuring, preferably PET imaging. ¹⁸FDG tracer is available worldwide and as a glucose analogue it may be incorporated by the erythrocytes and trapped intracellularly. In this sense, a blood sample (such as whole peripheral blood) can be extracted or isolated from a subject or from another subject with blood compatibility. The extracted or isolated blood can be then incubated in contact with ¹⁸FDG and later be reinjected into the patient for diagnostic purposes. Labeling of RBCs with ¹⁸FDG would expand the use of the PET technique to blood pool imaging with the consequent improvement in the field of clinical diagnosis.

Therefore, a first aspect of the invention refers to *in vitro* use of ¹⁸FDG (Fluorodeoxyglucose (18F)) for intracellular labeling red blood cells (RBCs). Such use overcomes the severe limitations that make blood pool measurements rarely accessible to patients or researchers.

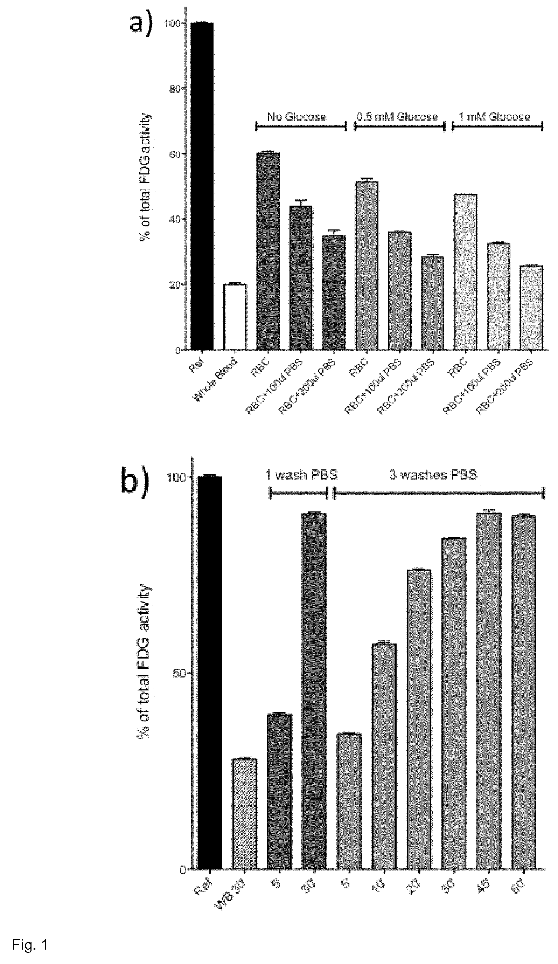

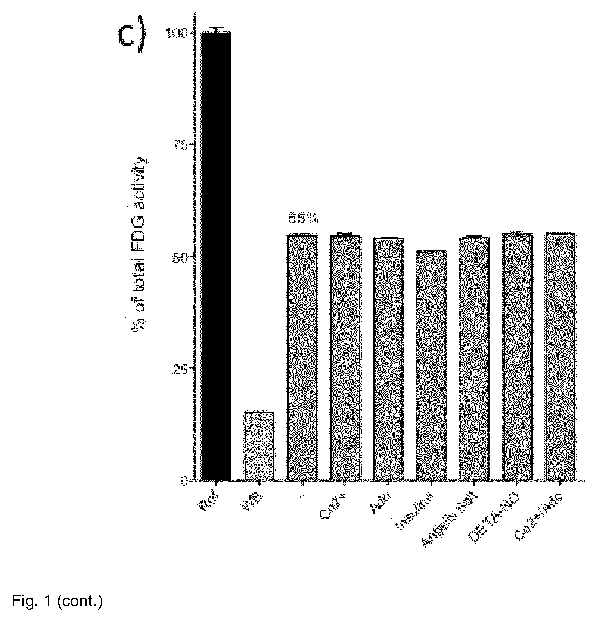

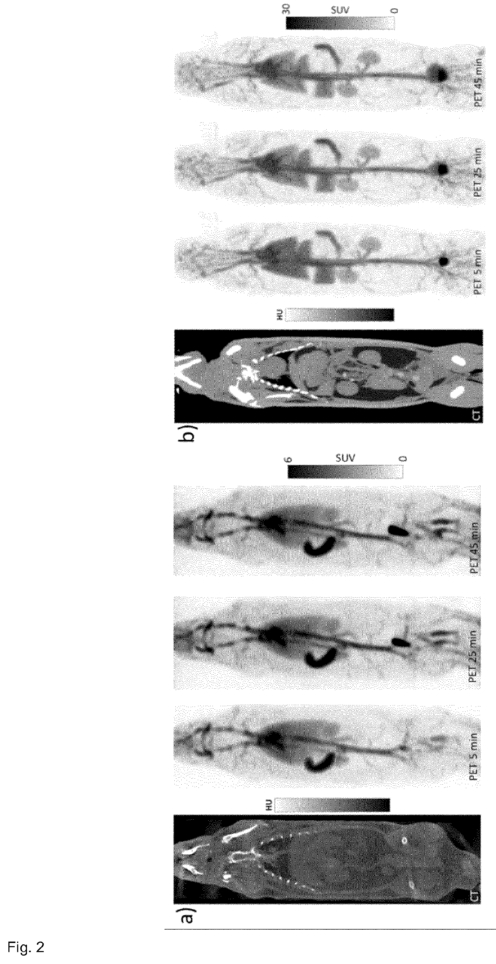

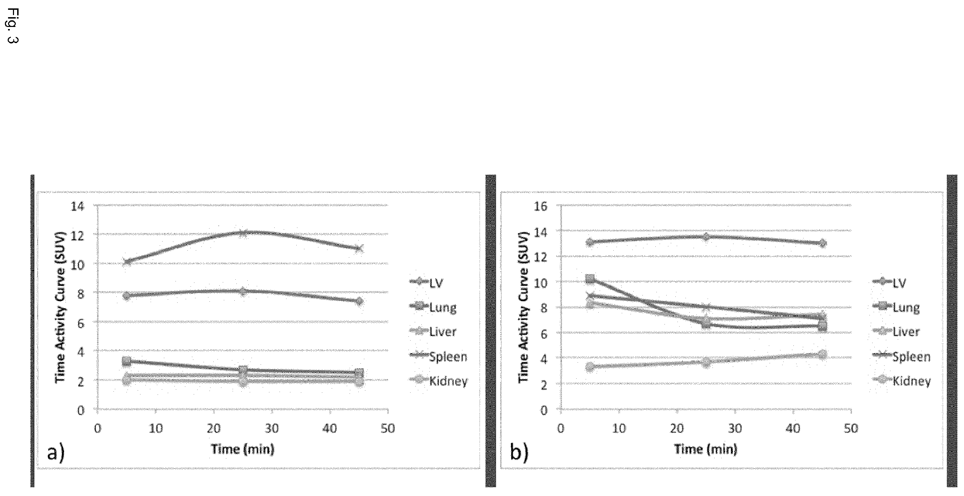

However, to practice the present invention it is noted that direct incubation of whole blood with ¹⁸FDG results in a poor uptake efficiency (see examples) of ¹⁸FDG by the RBCs, in fact, an insufficient uptake to use these labelled cells for blood pool imaging. In order to improve the uptake efficiency, we tested different strategies. In this sense, as shown in figure 2, we tested a number of different stimuli; however none of these stimuli affected the labeling efficiency as reflected in the examples. Yet, after numerous attempts we found an optimal labeling method that produced a 3-5 fold increased labeling efficiency (see figure 1) compared to direct incubation of whole blood with ¹⁸FDG. This optimum method was based on the removal of competing glucose from inside and outside the cells, such removal enhances the ¹⁸FDG membrane transport and metabolization. In addition, we also found that by minimizing the external volume of the RBCs prior to incubation with ¹⁸FDG and thus by compacting the cells, the ¹⁸FDG membrane transport to the interior of the cells increased. By using the aforesaid optimum methodology we were able, as shown in figure 3, to provide a very stable and reproducible distribution of activity in blood across time and animal species.

Therefore, in a preferred embodiment of the first aspect of the invention, the red blood cells are intracellularly glucose depleted red blood cells. Such depletion can be carried out, as shown in the examples, by re-suspending the RBCs in a saline buffer, such as PBS, in a ratio RBCs : saline buffer between 1:2 to 1:10, preferably in a ratio of about 1:5, preferably this step is performed one or more times, preferably one to three times, more preferably only once. Preferably, the glucose depleted red blood cells are contained within a glucose-depleted composition containing the RBCs. The RBCs' extracellular glucose present in the aforesaid composition can be removed by eliminating the plasma and buffy coat from the blood sample to a concentration of glucose present in the extracellular medium of less than 1mM, preferably less than 0.5 mM, more preferably to a concentration of about 0 mM.

A second aspect of the invention refers to a method for the intracellular labeling of red blood cells (RBCs), which comprises i) removing the extracellular glucose from a biological sample comprising RBCs isolated from a mammal, preferably from a human subject, and ii) removing the intracellular glucose present in the RBCs present in the aforesaid sample to obtain a glucose depleted red blood cells sample and/or generating a concentrated preparation of red blood cells that is obtained from step i) above after removing the plasma by centrifugation or by other sedimentation procedures; and contacting or mixing the glucose depleted red blood cells sample or the concentrated preparation of red blood cells with ¹⁸FDG. Preferably, the RBCs' extracellular glucose is removed by eliminating the plasma and buffy coat from the blood sample to a concentration of glucose present in the extracellular medium of less than 1mM, preferably less than 0.5 mM, more preferably to a concentration of about 0 mM. Also preferably, the glucose intracellular depletion can be carried out by re-suspending the RBCs in a saline buffer in a ratio RBCs : saline buffer between 1:2 to 1:10, preferably in a ratio of about 1:5, preferably this step is performed one or more times, preferably one to three times, more preferably only once.

In a preferred embodiment of the second aspect of the invention, the method for the intracellular labeling of red blood cells (RBCs), comprises the following steps:
a. Removing the RBCs' extracellular glucose from a biological sample containing the RBCs isolated from a mammal, preferably from a human subject;
b. Removing or depleting the intracellular glucose of the RBCs containing sample of step a);
c. Mixing the RBCs containing sample of step b) with ¹⁸FDG, wherein the ratio RBCs : ¹⁸FDG is greater than 1; and
d. Eliminating the unbound ¹⁸FDG;
wherein preferably,
a. the RBCs' extracellular glucose is removed by eliminating the plasma and buffy coat from the blood sample isolated from a mammal, preferably from a human subject;
b. the RBCs' intracellular glucose is depleted by re-suspending the RBCs containing sample of step a) in a saline buffer in a ratio RBCs : saline buffer less than 1;
c. the RBCs containing sample resulting from step b) is mixed with ¹⁸FDG in a ratio RBCs : ¹⁸FDG greater than 1;
wherein more preferably,
a. the RBCs' extracellular glucose is removed by eliminating the plasma and buffy coat from the blood sample isolated from a mammal, preferably from a human subject, by centrifuging the blood sample;
b. the RBCs' intracellular glucose is depleted by re-suspending the RBCs containing sample of step a) in a saline buffer in a ratio RBCs : saline buffer between 1:2 to 1:10, preferably in a ratio of about 1:5, preferably this step is performed only once; and
c. the RBCs containing sample resulting from step b) is mixed with ¹⁸FDG in a ratio from about 2:1 to 10:1,
wherein still more preferably,
the RBCs containing sample resulting from step b) has an external (extracellular) volume, preferably of a saline buffer such as PBS, of less than 200 µl, preferably less than 100 µl, more preferably less than 50 µl, still more preferably less than 10 µl, prior to the mixing step as detailed in step c). More preferably, such external (extracellular) volume of, preferably a buffer such as PBS, is between 0 and 5 µl prior to the mixing step detailed in step c),
and wherein still more preferably,
step b) is replaced by a new step b) wherein the RBCs containing sample resulting from step a) are treated so as to reduce the external (extracellular) volume of, preferably a buffer such as PBS, to less than 200 µl, preferably less than 100 µl, more preferably less than 50 µl, still more preferably less than 10 µl, or between 0 and 5 µl, prior to the mixing step detailed in step c).

In a preferred embodiment of the second aspect of the invention, the method comprises the following steps:
a. blood is withdrawn from a subject, preferably a human subject (typically 1-10 ml depending on the application);
b. Such blood is later centrifuged 10 minutes at 2000 x g at 4°C and the plasma and buffy coat (containing white cells and platelets) are discarded. In that way, most of the external glucose is removed;
c. Then, the RBCs are re-suspended in ice-cold phosphate buffer saline (PBS, 1:5, v/v) and gently mixed prior to an additional centrifugation. In this way, the glucose from inside the cells is transported outside into a much larger volume preventing it from reentering the cells. After centrifugation and removal of PBS the washed RBCs are ready for incubation with ¹⁸FDG. The internal and external glucose has been removed and the external volume has been minimized (packed cells);
d. ¹⁸FDG (100-500 MBq depending on the application) is mixed with the RBCs and incubated at 37°C for 30 min with gentle agitation. ¹⁸FDG is obtained from the cyclotron diluted in saline, typically at a concentration of 500 MBq/ml. The volume of ¹⁸FDG should be small in comparison with the volume of RBCs in order to produce optimal uptake efficiency.
e. After incubation, the RBCs are re-suspended in ice-cold PBS (1:2, v/v) and centrifuged in order to eliminate the unbound 18FDG. Optionally, this step can be repeated several times.

Finally, the FDG-labeled RBCs as obtained according to the second aspect of the invention or according to any of its preferred embodiments are re-suspended in saline, or PBS, or cell-free plasma (1:1, v/v) at room temperature. The resulting preparation is then ready to be injected to the patient or to an experimental animal model. Image acquisition protocols shall vary according to the particular application.

It is further noted that step a) of the methodology illustrated in the second aspect of the invention or in any of its preferred embodiments, could be performed by centrifugation as explained therein or alternatively using desalting columns, anyhow, if desalting columns are used it is strongly suggested to still use a centrifugation step in order to maximize the packing fraction of the RBCs.

A third aspect of the invention refers to red blood cells intracellularly labelled with ¹⁸FDG (Fluorodeoxyglucose (18F)) obtained or obtainable by the method of the second aspect of the invention or of any of its preferred embodiments. Alternatively, such intracellularly labelled with ¹⁸FDG (Fluorodeoxyglucose (18F)) could be further characterized by comprising a non-metabolized intracelular glucose concentration below 5 mM, preferably below 1 mM, more preferably below 0.5 mM, favouring the uptake upon ¹⁸FDG addition through the glucose transporters

A fourth aspect of the invention refers to the composition comprising the red blood cells as characterized in the third aspect of the invention.

A fifth aspect of the invention refers to the composition of the fourth aspect of the invention, for use in therapy or for use in *in vivo* diagnosis. Alternatively, the fifth aspect of the invention refers to a method of treatment or *in vivo* diagnosis by using the composition of the fourth aspect of the invention.

A sixth aspect of the invention refers to the composition of the fourth aspect of the invention, for use as a diagnostic radiopharmaceutical in *in vivo* blood pool imaging, preferably *in vivo* blood pool PET imaging.

A seventh aspect of the invention refers to a kit of parts comprising i) glucose depleted red blood cells contained within a glucose-depleted composition containing the RBCs; and ii) a ¹⁸FDG (Fluorodeoxyglucose (18F)) sample. Preferably, the ¹⁸FDG (Fluorodeoxyglucose (18F)) sample is diluted in a solvent at a concentration of between 100 to 500 MBq/mL.

An eighth aspect of the invention refers to a method for *in vivo* blood pool imaging comprising the use of Red blood cells intracellularly labelled with ¹⁸FDG (Fluorodeoxyglucose (18F)), preferably obtained or obtainable by the method of any of claims 5 to 7. Preferably, such method is for *in vivo* blood pool PET imaging.

A ninth aspect of the invention refers to the use of Red blood cells intracellularly labelled with ¹⁸FDG (Fluorodeoxyglucose (18F)) as an *in vivo* diagnostic radiopharmaceutical.

### EXAMPLES

### Materials and Methods

We divided the study in two different steps. In a first step we performed an *in vitro* study to compare different labeling protocols and a second step to evaluate the optimum protocol in different animal species. The animal protocols were approved by the Ethics Committee for Animal Experimentation of the Comunidad de Madrid. *In vitro* studies were performed with blood from New Zealand white male rabbits. Two New Zealand white male rabbits (mean weight 4.9±0.2 kg; Charles River), and one Wistar rat (430 g) were used for *in vivo* blood pool PET imaging studies. The rabbit studies were performed on a Philips Gemini TF PET/CT systems and the rat study on a Mediso NanoPET/CT system. The details about the animal handling and image protocol are summarized on table 2 below. CT images were acquired prior to CT images for anatomical coregistration and to perform attenuation and scatter corrections. Whole body PET acquisitions were performed starting at 5, 25 and 45 minutes after dose injection in order to study the bio-distribution at different time points. For that purpose, the images were loaded into Oxirix (Pigmeo) software and 2D regions of interest (ROIs) were drawn on sagittal views in order to compute the mean activity distribution in different organs (left ventricle, lung, liver, spleen and kidney) in standardized uptake value (SUV) units.

**Table 2. Animal preparation and imaging protocols used for in vivo blood pool PET imaging in rats and rabbits.**

| **Animal** | **Rat** | **Rabbit** |
|---|---|---|
| **Anesthesia** | Isoflurane (5% induction, 2% maintenance), 1% oxygen | ketamine (30 mg/kg; Fort Dodge Animal Health) and xylazine (5 mg/kg; Bayer Corp) |
| **Blood volume (ml)** | 1 | 5 |
| **Sample site** | Tail vein | Marginal ear artery |
| **Animal position** | Prone | Prone |
| **CT protocol** | Helical scan, 60 kV | Helical, 120 kV |
| **Injected dose (MBq)** | 37 | 32 |
| **Injection site** | Tail vein | Marginal ear vein |
| **Number of bed positions** | 4 | 5 |
| **Acquisition time (min/bed)** | 2.5 | 2 |
| **PET acquisition start time since injection (min)** | 5,25,45 | 5,25,45 |
| **PET reconstruction protocol** | 3D-OSEM, Full detector model Voxel size: 0.6×0.6×0.6 mm³ | 3D-RAMLA, 3 iterations 33 subsets. Voxel size: 2×2×2 mm³ |

### Example 1. Results

### 1.1 In vitro studies

By direct incubation of whole blood with ¹⁸FDG only 10-20% of the ¹⁸FDG was bound to the cells within a reasonable period time (30-60 minutes, compared to the half live of the isotope ¹⁸F, 109 minutes). In order to improve the uptake efficiency, we tested several strategies. One of these strategies consisted in promoting ¹⁸FDG consumption by removing all competing glucose from inside and outside the RBCs. Further strategies consisted in adding particular stimuli to the blood that could potentially increase glucose consumption.

### - Glucose depletion

In order to increase the transport of ¹⁸FDG across the cell membrane we proposed removing all the glucose outside the cell and to minimize the external volume. In that way, we eliminated any competition with the labeled glucose via the glucose transporters (removing external glucose) and we in addition increased the probability of ¹⁸FDG contacting the cells by minimizing the external volume.

However, such actions were not sufficient since once the ¹⁸FDG is inside the cell it must be metabolized to ¹⁸FDG-6-phosphate in order to remain trapped, otherwise it can be transported out of the cell again. However, inside the cell there is a large amount of glucose (millimolar concentration) in comparison to the amount of glucose that can be metabolized during de incubation period and much larger than the amount of ¹⁸FDG available (picomolar concentration). Therefore, our methodology combines the extracellular removal of glucose with the removal of glucose from inside the cells prior to cell incubation with ¹⁸FDG. In summary, the proposed methodology enhances both the transport of FDG inside the cells and its conversion into ¹⁸FDG-6-phospate to be trapped intracellularly.

To illustrate such methodology in the present invention, we performed a series of *in vitro* studies with different RBCs packing fractions and glucose levels. For this purpose, all blood samples were centrifuged to separate plasma and buffy coat from RBCs and washed three times with PBS (1:2, v/v). In order to test different RBCs packing fractions these were re-suspended with different amounts of PBS (1:0 = no PBS, 1:1 and 1:2; v/v) and each case was tested with different glucose concentration (0, 0.5 and 1 mM). Duplicates of each sample were prepared, mixed with the same amount of ¹⁸FDG (1:0.05, v/v) and incubated for 30 minutes at 37ºC. An additional study was performed to study the labeling efficiency for different incubation times (5, 10, 20, 30, 45 and 60 minutes). Furthermore, to test the effect of glucose depletion we designed a protocol consisting in one washing step with PBS (1:5, v:v) before the incubation step.

The steps followed to implement the optimized protocols were:
1.- Blood was withdrawn from the patient or the experimental animal model under study (typically 1-10 ml depending on the application).
2.-The blood was centrifuged 10 minutes at 2000 x g at 4°C and the plasma and buffy coat (containing white cells and platelets) were discarded. In that way, most of the external glucose was removed.
3.- The RBCs were re-suspended in ice-cold phosphate buffer saline (PBS, 1:5, v/v) and gently mixed prior to an additional centrifugation. In this way, the glucose from inside the cells was transported outside into a much larger volume preventing it from reentering the cells. After centrifugation and removal of PBS the washed RBCs were ready for incubation with ¹⁸FDG. The internal and external glucose were removed and the external volume was minimized (packed cells).
4.-The ¹⁸FDG (100-500 MBq depending on the application) was mixed with the RBCs and incubated at 37°C for 30 min with gentle agitation. ¹⁸FDG was obtained from the cyclotron diluted in saline, typically at a concentration of 500 MBq/ml.
5.- After incubation, the RBCs were re-suspended in ice-cold PBS (1:2, v/v) and centrifuged in order to eliminated the unbound ¹⁸FDG. Optionally, this step can be repeated several times.
6.- Finally, the FDG-labeled RBCs were re-suspended in saline, or PBS, or in cell-free plasma (1:1, v/v) at room temperature. The resulting preparation was then ready to be injected to a patient or to an experimental animal model.

### - Activation of the glucose transport through the RBC membrane and cell metabolism

The transfer rate of glucose across the RBC membrane can be affected in many different ways. Cells possessing low glucose transport capacity relative to sugar metabolic capacity are generally susceptible to acute regulation of glucose transport. Those cells are modulated rapidly by a variety of stimuli, including insulin, anoxia, and metabolic poisons. Nonetheless, tissues possessing high transport capacity relative to metabolic capacity (nerve and human erythrocytes) can also respond to a number of stimuli with altered glucose transport rates. In addition, glucose is transferred inside and outside the cells continuously by the glucose transporters. However, ¹⁸FDG will only be trapped within the cell if it is metabolized and converted into ¹⁸FDG-6-phosphate. Therefore, a logical manner to increase the labeling efficiency was to try to accelerate the cell metabolism. In particular, we tested the following stimuli: adenosine, insulin, cobalt, nitric oxide donors (DETA-NO) nitroxyl donors (Angeli's salt) and a combination of adenosine and cobalt. The different stimuli were tested using the optimal protocol described above.

Figure 1 shows the percentage of ¹⁸FDG trapped into the RBCs under different preparation conditions. Figure 1.a demonstrates a three-fold efficiency increase for the case of maximum packing of RBCs and no addition of glucose compared to whole blood labeling. The labeling efficiency was decreased for higher added concentrations of glucose and reduced RBCs packing fractions. The labeling efficiency at different incubation times is shown in figure 1.b. We also tested two different washing strategies to produce glucose-free packed RBCs (one vs. three washes of packed cells). Almost 100% of the ¹⁸FDG was bound after 45 minutes of incubation.

The preparation protocol followed in all cases was the optimal one shown in figure 1.a, i.e. no added glucose and maximum RBCs packing fraction. The strategy of washing cells only once with a larger amount of PBS produced similar results than washing three times the RBCs in PBS (1:1, v/v). Figure 1.c shows the efficiency results obtained for different stimuli after 10 minutes of incubation. Minor differences compared to the non-stimuli case were observed. Therefore, unfortunately none of the stimuli used provided for a solid methology to improve the uptake efficiency of ¹⁸FDG.

### 1.2 In vivo studies

Figure 2 shows CT and PET images obtained at different time points after dose injection. Small variations of activity distribution at different time point for both rat and rabbit studies except for the bladder that shows increase activity from washed ¹⁸FDG. Figure 3 shows the quantitative measurement performed on different organs. The concentration of activity in blood (left ventricle) remains constant during the first hour after dose injection. Increased lung activity concentration is observed in the rabbit's lung 5 minutes after dose injection which is later reduced.

## Claims

1. *In vitro* use of ¹⁸FDG (Fluorodeoxyglucose (18F)) for intracellular labeling red blood cells (RBCs).

2. The *in vitro* use of claim 1, wherein the red blood cells are glucose depleted red blood cells having a concentration of non-metabolized intracelular glucose below 1 mM.

3. The *in vitro* use of any of claims 1 or 2, wherein the RBCs are comprised in an external or extracellular volume of less than 200 µl.

4. The *in vitro* use of any of claims 2 to 3, wherein the glucose depleted red blood cells or the RBCs are comprised within a glucose-depleted composition containing the RBCs.

5. A method for the intracellular labeling of red blood cells (RBCs), which comprises i) removing the extracellular glucose from a biological sample comprising RBCs isolated from a mammal, preferably from a human subject, and ii) removing the intracellular glucose present in the RBCs present in the aforesaid sample to obtain a glucose depleted red blood cells sample and/or reducing the external (extracellular) volume of less than 200 µl; and contacting or mixing the red blood cells sample with ¹⁸FDG.

6. A method for the intracellular labeling of red blood cells (RBCs), which comprises:
a. Removing the RBCs' extracellular glucose from a biological sample containing the RBCs isolated from a mammal;
b. Removing or depleting the intracellular glucose of the RBCs containing sample of step a);
c. Mixing the RBCs containing sample of step b) with ¹⁸FDG, wherein the ratio RBCs : ¹⁸FDG is greater than 1; and
d. Eliminating the unbound ¹⁸FDG.

7. The method of claim 6, wherein:
a. the RBCs' extracellular glucose is removed by eliminating the plasma and buffy coat from the blood sample isolated from a mammal, preferably from a human subject;
b. the RBCs' intracellular glucose is depleted by re-suspending the RBCs containing sample of step a) in a saline buffer in a ratio RBCs : saline buffer less than 1;
c. the RBCs containing sample resulting from step b) is mixed with ¹⁸FDG in a ratio RBCs : ¹⁸FDG greater than 1; and
d. Eliminating the unbound ¹⁸FDG.

8. The method of claim 6, wherein:
a. the RBCs' extracellular glucose is removed by eliminating the plasma and buffy coat from the blood sample isolated from a mammal, preferably from a human subject, by centrifuging the blood sample;
b. the RBCs' intracellular glucose is depleted by re-suspending the RBCs containing sample of step a) in a saline buffer in a ratio RBCs : saline buffer between 1:2 to 1:10, preferably in a ratio of about 1:5, preferably this step is performed only once;
c. the RBCs containing sample resulting from step b) is mixed with ¹⁸FDG in a ratio from about 2:1 to 10:1; and
d. Eliminating the unbound ¹⁸FDG.

9. The method of any of claims 6 to 8, wherein the RBCs containing sample resulting from step b) has an external (extracellular) volume of less than 200 µl, prior to the mixing step detailed in step c).

10. The method of any of claims 6 to 8, wherein RBCs containing sample resulting from step b) has an external (extracellular) volume of between 0 and 5 µl, prior to the mixing step detailed in step c).

11. The method of any of claims 6 to 8, wherein step b) is replaced by a new step b) wherein the RBCs containing sample resulting from step a) are treated so as to reduce the external or extracellular volume to less than 200 µl, prior to the mixing step detailed in step c).

12. Red blood cells labelled with ¹⁸FDG (Fluorodeoxyglucose (18F)) obtained or obtainable by the method of any of claims 6 to 11.

13. A composition comprising the red blood cells of claim 12.

14. A composition according to claim 11, for use in therapy or for use in *in vivo* diagnosis.

15. A composition according to claim 13, for use as a diagnostic radiopharmaceutical in *in vivo* blood pool imaging, preferably *in vivo* blood pool PET imaging.

16. A kit of parts comprising i) glucose depleted red blood cells contained within a glucose-depleted composition containing the RBCs; and ii) a ¹⁸FDG (Fluorodeoxyglucose (18F)) sample.

17. The kit of parts of claim 16, wherein the ¹⁸FDG (Fluorodeoxyglucose (18F)) sample is diluted in a solvent at a concentration of between 100 to 500 MBq/mL.
